(19) Europäisches Patentamt
European Patent Office
Office européen des brevets

(11) **EP 4 511 672 B1**

(12) **EUROPEAN PATENT SPECIFICATION**

(45) Date of publication and mention of the grant of the patent:
**25.03.2026 Bulletin 2026/13**

(21) Application number: **23722780.6**

(22) Date of filing: **18.04.2023**

(51) International Patent Classification (IPC):
***G01R 33/565*** *(2006.01)* ***G01R 33/48*** *(2006.01)*
***G01R 33/561*** *(2006.01)* ***G01R 33/24*** *(2006.01)*

(52) Cooperative Patent Classification (CPC):
**G01R 33/56554;** G01R 33/243; G01R 33/4806; G01R 33/4822; G01R 33/5611

(86) International application number:
**PCT/US2023/018940**

(87) International publication number:
**WO 2023/205143 (26.10.2023 Gazette 2023/43)**

(54) **ECHO-SHIFTED ECHO-PLANAR IMAGING WITH SIMULTANEOUS BLIP-UP AND BLIP-DOWN ACQUISITIONS FOR CORRECTING GEOMETRIC DISTORTION**

ECHOVERSCHOBENE ECHOPLANARBILDGEBUNG MIT GLEICHZEITIGEN BLIPUP- UND BLIPDOWN-ERFASSUNGEN ZUR KORREKTUR GEOMETRISCHER VERZERRUNGEN

IMAGERIE ÉCHO-PLANAIRE À DÉCALAGE D’ÉCHO À ACQUISITIONS SIMULTANÉES D’ENCODAGE DE PHASE DIRECTE ET INVERSÉE (BLIP-UP ET BLIP-DOWN) POUR LA CORRECTION D’UNE DISTORSION GÉOMÉTRIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **18.04.2022 US 202263332260 P**

(43) Date of publication of application:
**26.02.2025 Bulletin 2025/09**

(73) Proprietor: **The Board of Trustees of the University of Illinois**
**Urbana, IL 61801 (US)**

(72) Inventors:
- **ZHOU, Xiaohong Joe**
  **Naperville, Illinois 60563 (US)**
- **SUN, Kaibao**
  **Chicago, Illinois 60612 (US)**

(74) Representative: **Rentsch Partner AG**
**Kirchenweg 8**
**Postfach**
**8034 Zürich (CH)**

(56) References cited:
**US-B2- 10 429 476**

- **CONGYU LIAO ET AL: "Highly efficient MRI through multi-shot echo planar imaging", ARXIV.ORG, CORNELL UNIVERSITY LIBRARY, 201 OLIN LIBRARY CORNELL UNIVERSITY ITHACA, NY 14853, 2 August 2019 (2019-08-02), XP081455169**
- **KAIBAO SUN ET AL: "Three-dimensional echo-shifted echo planar imaging with simultaneous blip-up and blip-down acquisitions for correcting geometric distortion", PROCEEDINGS OF THE JOINT ANNUAL MEETING ISMRM-ESMRMB 2022, no. 4390, 22 April 2022 (2022-04-22), London, XP040730938**
- **XIAOZHI CAO ET AL: "Efficient T2 mapping with blip-up/down EPI and gSlider-SMS (T2-BUDA-gSlider)", MAGNETIC RESONANCE IN MEDICINE, WILEY-LISS, US, vol. 86, no. 4, 28 May 2021 (2021-05-28), pages 2064 - 2075, XP071675391, ISSN: 0740-3194, DOI: 10.1002/MRM.28872**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BILGIC B. ET AL.: "3D-BUDA Enables Rapid Disortion-Free QSM Acquisition", PROCEEDINGS OF THE INTERNATIONAL SOCIETY FOR MAGNETIC RESONANCE IN MEDICINE, no. 596, 24 July 2020 (2020-07-24), XP040714511**

## Description

### STATEMENT OF GOVERNMENTAL INTEREST

This invention was made with awards 5R01EB026716 and 1S10RR028898 from the National Institute of Health (NIH). The government has certain rights in the invention.

### BACKGROUND

Blood oxygenation level-dependent (BOLD) functional MRI (fMRI) is a primary technique for mapping neural activity of the human brain. Human brain mapping using MRI is typically performed using a set of two-dimensional axial slices. However, three-dimensional (3D) acquisitions using gradient-echo echo-planar imaging (GRE-EPI) are gaining momentum due to their fast acquisition speed, robustness against motion, whole-brain spatial coverage, and high signal-to-noise ratio (SNR) per unit time. In 3D GRE-EPI, conventional phase-encoding with a stepping gradient is employed in one spatial dimension (e.g., the z-axis or the "slice" direction), while EPI-type phase encoding with blip gradients is applied to the second spatial dimension (e.g., the y-axis or the phase direction) and EPI-type bipolar readout gradient is used to spatially encode the third dimension. However, since the sampling bandwidth is narrow (e.g., < 2 kHz) in the blipped phase-encoding direction, severe geometric distortions can occur in the presence of magnetic field inhomogeneities and other off-resonance effects. If the associated perturbing field gradient has the same polarity as the blip phase-encoding gradient, then the images are stretched along the phase-encoding direction. Conversely, if the gradient polarities are opposite, then the images are compressed.

Several distortion correction techniques have been proposed for EPI images over the past decades. One approach is to correct distortions in the image domain using a $B_0$-field map, which is obtained from a double-echo gradient echo ("GRE") image. The method can effectively correct signal-stretching artifacts in the images. However, an EPI acquisition with only one phase-encoding polarity does not provide sufficient information to correct for signal pile-up in regions with substantial off-resonance effects, where signals from different locations are compressed into a single pixel and hence their spatial information is lost. To address this issue, image-domain registration based on two EPI data sets with opposite phase-encoding directions (e.g., TOPUP in FSL) have been developed, and adopted in several large-scale neuroimaging studies. Nonetheless, the registration errors may fail distortion correction when the image SNR is poor, especially in the anterior temporal lobe and orbitofrontal cortex. This issue has been addressed using a technique known as BUDA (blip-up/down acquisition). BUDA is based on a forward model that links the distortion-free image to the corrupted raw k-space data. This allows distortion-corrected images to be reconstructed from the k-space data acquired with blip-up and blip-down phase-encoding gradients by solving the corresponding inverse problem with Hankel structured low-rank constraint. In previous studies, BUDA has been successfully applied to susceptibility, diffusion and relaxation-weighted imaging and demonstrated its robustness in geometric distortion correction for two-dimensional ("2D") and 3D EPI.

Despite its excellent performance, BUDA requires two separate acquisitions (or shots) with blip-up and blip-down, respectively, which compromises the imaging efficiency. A multi-shot echo-planar imaging (EPI) method with blip-up and blip-down acquisitions is disclosed in Congyu Liao et al., "Highly efficient MRI through multi-shot echo planar imaging", arXiv:1908.00280, Cornell University Library, 2 August 2019. In this approach, two interleaved EPI shots are acquired with opposite phase-encoding blip gradient polarities to enable geometric distortion correction. However, this method requires two separate excitations, or shots, which compromises temporal resolution and increases scan time. This can be particularly problematic for fMRI because the need for two acquisitions halves the temporal resolution for resolving BOLD signal changes. Moreover, the longer scan times can also impose a physiological burden to the subject and increase vulnerability to motion. Very recently, an echo-shifting technique was employed to incorporate two EPI echo-trains with the same phase-encoding gradient polarity into a single sequence to increase the time efficiency for 2D EPI. This approach, however, is subject to a serious SNR loss caused by large flip angles needed in 2D multi-slice acquisitions. For example, only ~50% SNR can be retained under the optimal condition when compared to conventional 2D EPI with a 90° flip angle.

### SUMMARY

Systems, methods, and computer readable mediums for correcting geometric distortion in echo-planar magnetic resonance imaging are set forth herein. A method in accordance with the invention is defined in claim 1. Claims 2-9 specify preferred embodiments. The echo planar imaging ("EPI") sequence is widely used in magnetic resonance imaging ("MRI") due to its fast acquisition speed and robustness against motion. This method, however, is subject to geometric distortion caused by various off-resonance effects, including magnetic field inhomogeneities and magnetic susceptibility variations. Although the issue can be addressed by acquiring two k-space data sets with the opposite phase-encoding gradient polarity (i.e., blip-up/-down acquisition or BUDA) followed by combining the datasets to estimate a field map for distortion correction, a disadvantage is the doubled scan time due to the need of the two separately acquired datasets. Exemplary implementations disclosed herein integrate the two acquisitions into a single acquisition (or single shot) by using a novel echo-shifting technique. This method or technique is referred to herein as "Echo-shifted EPI with blip-up and -down acquisitions ('esEPI-BUDA')."

In accordance with the principles disclosed herein, esEPI-BUDA consists of two radiofrequency ("RF") pulses in one repetition time (or one shot), followed by two successive echo-trains with polarity-reversed phase-encoding gradients. A set of "echo-shifting gradient pulses" can be employed to allow the two echo-trains to acquire the signals from the first and second RF pulses, respectively. Thus, esEPI-BUDA can beneficially halve the total scan time of the conventional BUDA method and minimize inconsistent phase variations due to subject motion between the two acquisitions. By integrating the two acquisitions into one shot followed by joint image reconstruction, the exemplary methods herein can produce distortion-free images without increasing the scan times and without being subject to patient motion. This exemplary technique is demonstrated herein on phantoms and human subjects.

In a first aspect of the disclosure, an example method for using an MRI system electrically coupled to a computing device is disclosed. The method includes (a) generating, via the MRI system, an echo-shifted echo-planar imaging with blip up/down acquisition ("esEPI-BUDA") pulse sequence comprising a first radiofrequency ("RF") pulse and a second RF pulse, the first RF pulse followed by a first echo-train that is interleaved with the first and the second RF pulses, and the second RF pulse followed by a second echo-train such that the first and the second echo-trains have opposite phase-encoding blip gradient polarities to traverse echo planar imaging ("EPI") k-space in a reversed order, (b) in response to the pulse sequence being generated, the MRI system acquiring two k-space datasets within a single shot, and (c) correcting image distortion, via the MRI system, based on the two acquired k-space datasets.

A second aspect of the invention concerns a non-transitory computer-readable medium in accordance with claim 10.

The features, functions, and advantages that have been discussed can be achieved independently in various examples or may be combined in yet other examples further details of which can be seen with reference to the following description and drawings.

## BRIEF DESCRIPTION OF THE DRAWINGS

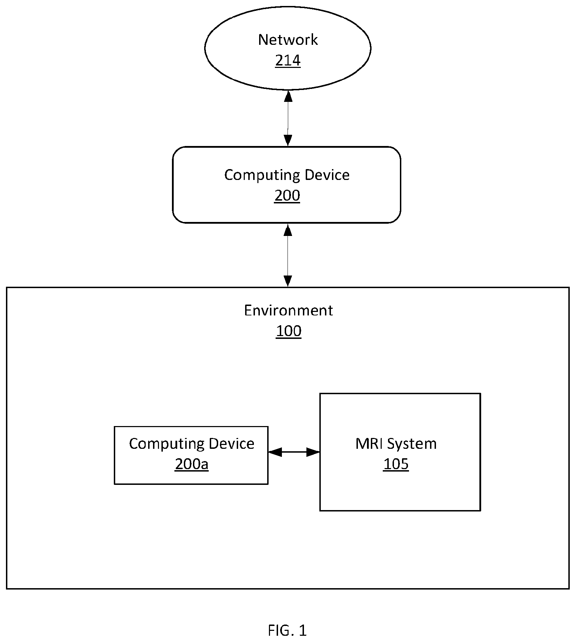

Figure 1 is a functional block diagram of an MRI system electrically coupled to a computing device, according to one example implementation;

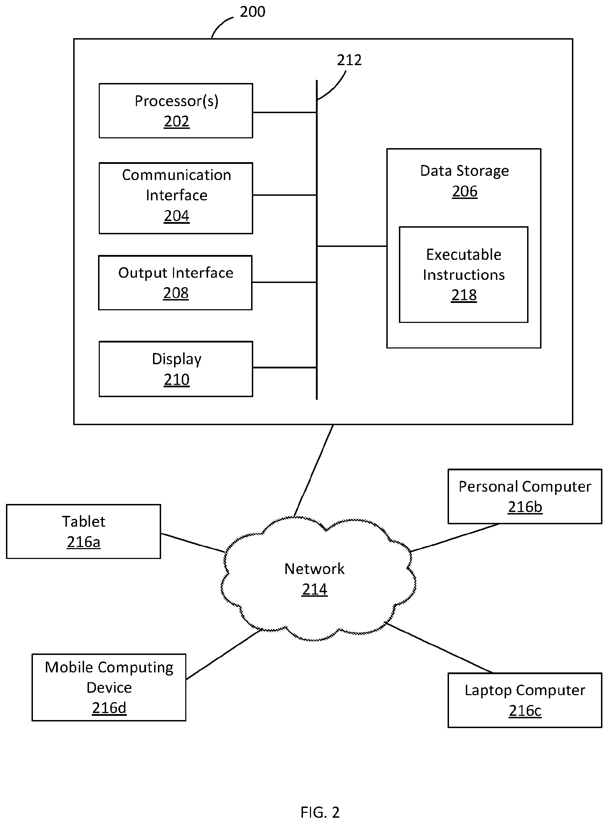

Figure 2 depicts a block diagram of a computing device and a computer network, according to an example implementation;

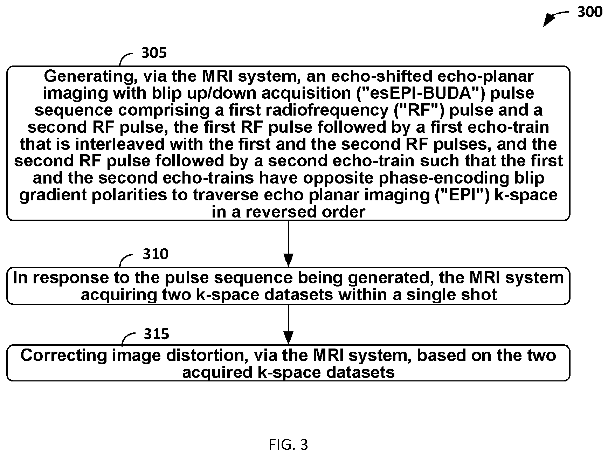

Figure 3 shows a flowchart of a method for using the MRI system, according to an example implementation;

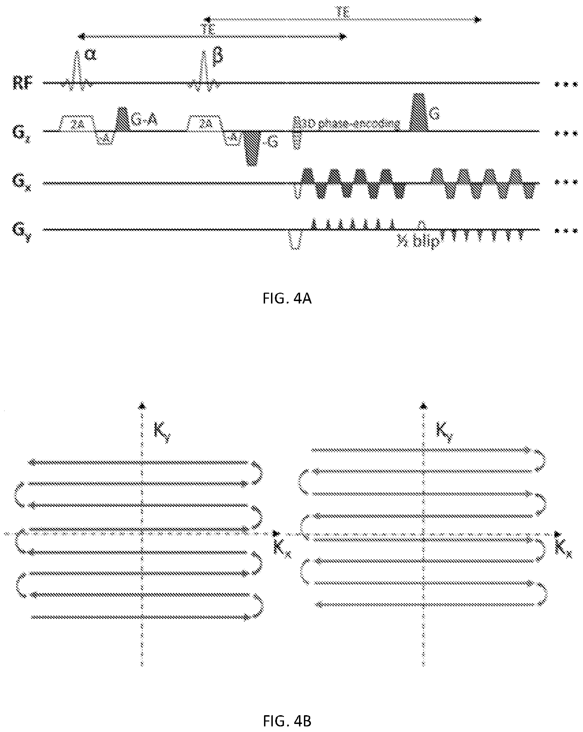

Figures 4A-B depict a schematic illustrating 3D echo-shifted EPI with blip-up and blip-down acquisitions ("esEPI-BUDA") in a single repetition time ("TR") (Figure 4A), and the corresponding two k-space trajectories (Figure 4B). Echo-shifting gradients (with areas of ($G - A$), $-G$, and $G$ as indicated in the figure) are applied along the slab-selection direction ($G_z$) to select the signals for the two echo-train acquisitions (black on the lefthand side and gray on the righthand side) from the first and second RF pulses, respectively. A small gradient with ½ phase-encoding blip area ($G_y$) is played out prior to the second echo-train so that the two k-space trajectories are interleaved, according to one example implementation;

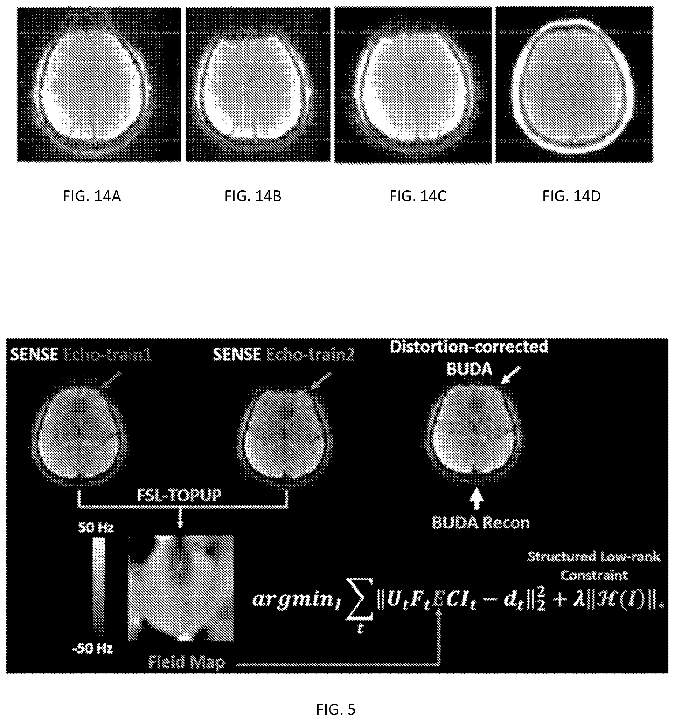

Figure 5 depicts the image reconstruction steps involved in esEPI-BUDA by using the human brain as an example. Each under-sampled echo-train dataset (i.e., Echo-train 1 with blip-up acquisition and Echo-train 2 with blip-down acquisition) first underwent 3D SENSE reconstruction individually, followed by TOPUP in FSL to estimate a field map E. The field map was subsequently incorporated to jointly reconstruct the data from both echo-trains with Hankel structured low-rank regularization for geometric distortion correction, according to one example implementation;

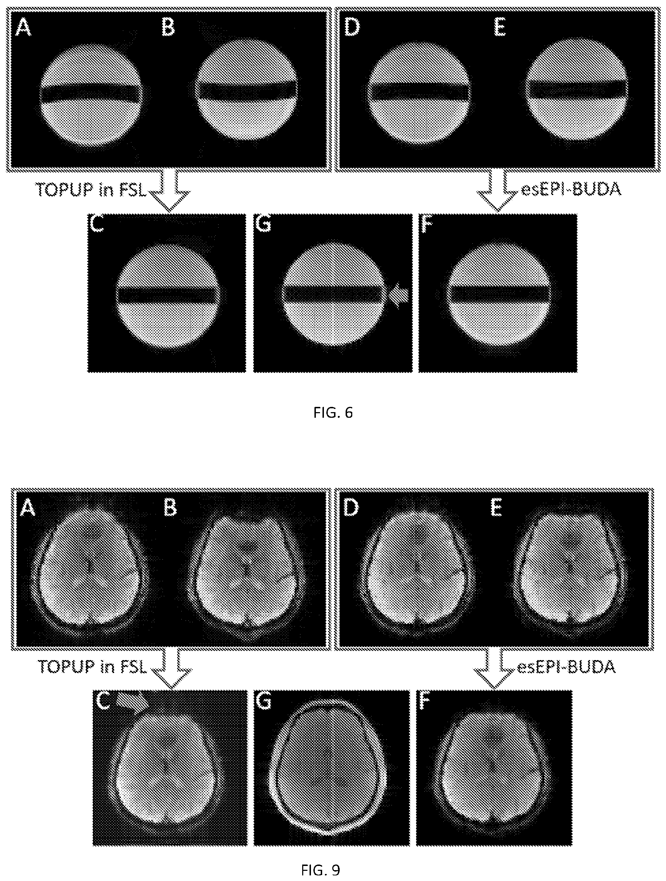

Figure 6 depicts representative images of a slice selected from the 3D datasets of DQA phantom acquired using 3D fully-sampled EPI with separate blip-up (A) and blip-down (B) acquisitions, 3D EPI TOPUP (C), 3D esEPI-BUDA (SENSE EPI in Echo-train 1 (D); SENSE EPI in Echo-train 2 (E); esEPI-BUDA (F)), and 3D SPGR (G). Using the horizontal black block (low-signal area indicated by the arrow in (G) as a fiducial mark, the images in (A) and (B) exhibited excessive image distortion that was reduced in (D) and (E). Image distortion was substantially corrected in (C) and (F). Image (C) shows that the geometric distortion was corrected by image registration of two 3D fully-sampled EPI (A) and (B) images using TOPUP in FSL. Image (F) shows that 3D esEPI-BUDA produced distortion-corrected images by jointly reconstructing two interleaved k-space datasets with reverse filling trajectories. Using the 3D SPGR image as a reference, the esEPI-BUDA image exhibited a higher degree of similarity with a SSIM of 0.91 and a lower NRMSE of 0.06, than the 3D EPI TOPUP image with a SSIM of 0.87 and a NRMSE of 0.08, according to one example implementation;

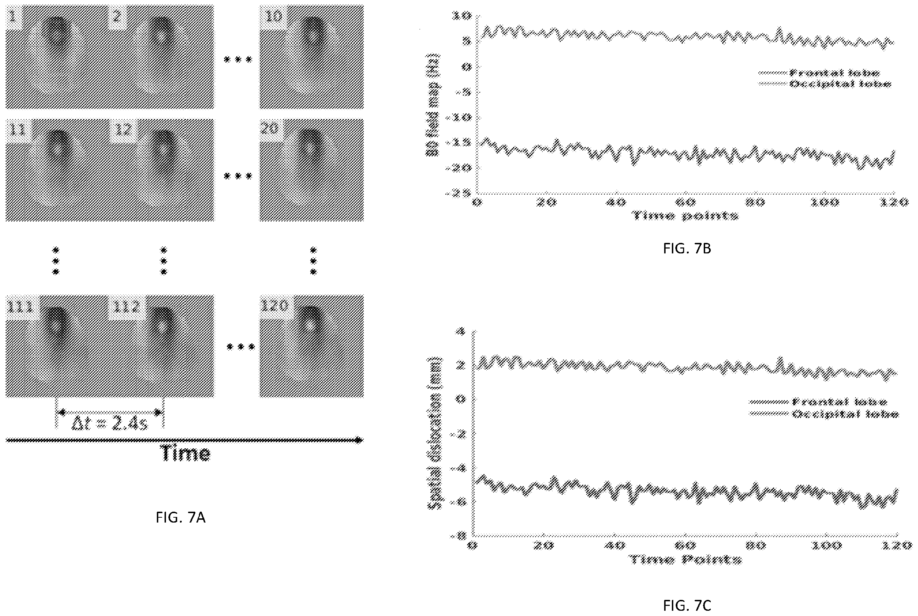

Figure 7A depicts a set of 120 $B_0$ maps in a slice randomly selected from the 3D datasets of the human brain, covering a total time span of 4 min and 48 sec with a temporal resolution of 2.4 sec;

Figure 7B depicts the dynamic $B_0$ evolutions at the frontal (top box of Figure 7A) and occipital (bottom box ofFigure 7B) lobes, according to one example implementation;

Figure 7C depicts the corresponding spatial $\Delta d$ dislocation caused by $B_0$. As shown in Figures 7B-7C, the maximal $B_0$ shifts during the process were approximately 6.2 Hz and 4.7 Hz in the two brain regions, corresponding to the spatial dislocations of 1.95 mm and 1.47 mm, respectively, according to one example implementation;

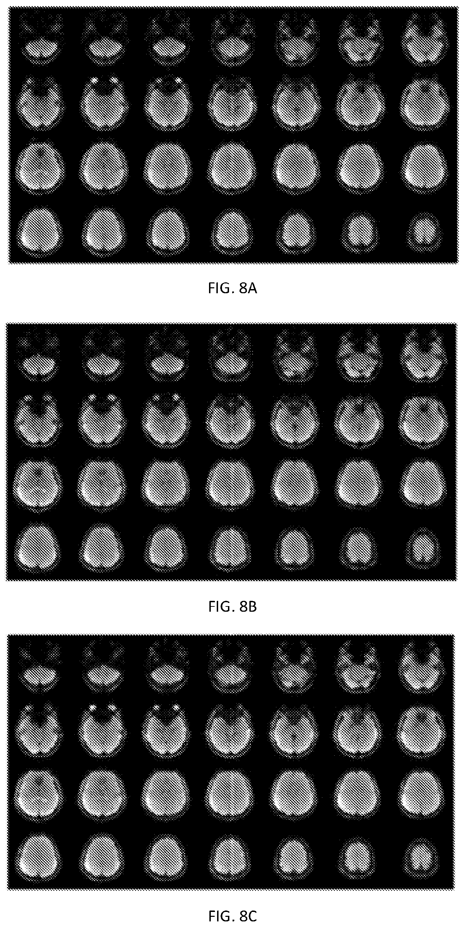

Figures 8A-C depict representative whole-brain 3D images from a healthy human subject (SENSE EPI in Echo-train 1 (Figure 8A), SENSE EPI in Echo-train 2 (Figure 8B), esEPI-BUDA (Figure 8C)) obtained in one volume TR using the 3D esEPI-BUDA sequence. Compared with the SENSE reconstruction using the blip-up or blip-down data, esEPI-BUDA image reconstruction allowed for a joint reconstruction of blip-up and blip-down data and produced improved image quality with the least image distortion, particularly in the areas with large magnetic susceptibility variations, such as the frontal lobe, according to one example implementation;

Figure 9 depicts representative images of a slice with a voxel size of $3.1 \times 3.1 \times 4$ mm$^3$randomly selected from the 3D datasets of the human brain. Images (A) and (B) were acquired using 3D fully-sampled EPI with separate blip-up (A) and blip-down (B) acquisitions. Image (C) depicts an image reconstructed from images (A) and (B) by using 3D EPI TOPUP. Images (D) and (E) were reconstructed from the first and second echo train of the esEPI-BUDA sequence, respectively. Image (F) shows the resultant esEPI-BUDA image reconstructed from both echo trains. Image (G) displays a conventional 3D SPGR image. Using the 3D SPGR image with a short TE (1.4 ms) as a reference, the images in (A) and (B) exhibited excessive image distortion, which was reduced in images (D) and (E). Image distortion was effectively corrected in images (C) and (F), according to one example implementation;

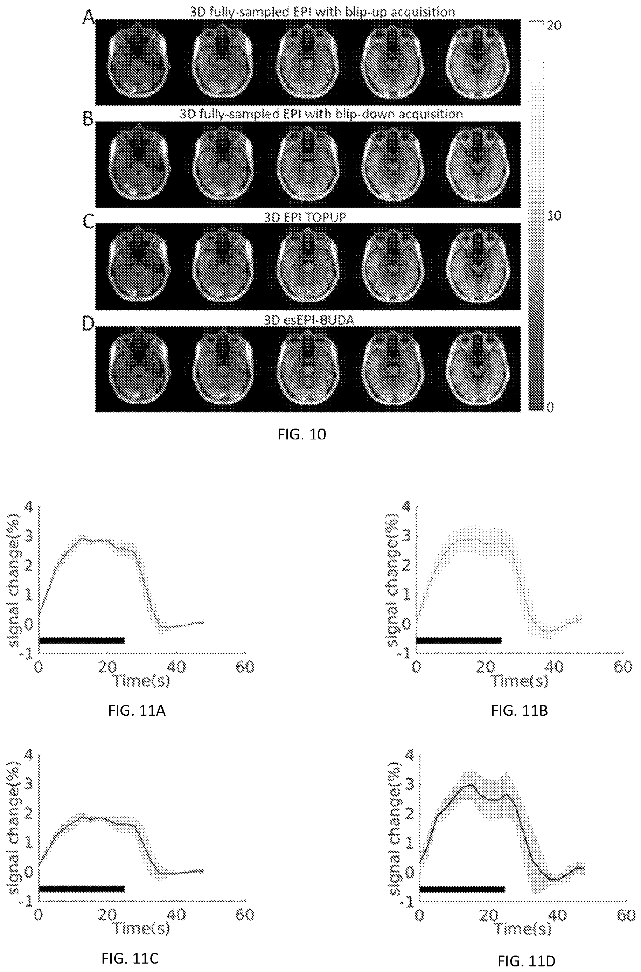

Figure 10 depicts representative visual fMRI activation maps of 3D fully sampled EPI with separated blip-up (Row A) and blip-down (Row B) acquisitions, the corresponding 3D EPI TOPUP (Row C), and 3D esEPI-BUDA (Row D), overlaid onto the T1-weighted images that are co-registered with 3D SPGR images. The activation maps co-register better with brain parenchyma in the T1-weighted structure images for 3D esEPI-BUDA, compared with conventional 3D fully-sampled EPI and the corresponding 3D EPI TOPUP images. These functional activation maps were rendered in color in the priority document and more fully depict the contrast between the images, according to one example implementation;

Figures 11A-D depict averaged time course with standard deviations (across the six subjects) for the images from 3D fully sampled EPI with separate blip-up (Figure 8A) and blip-down (Figure 11B) acquisitions, the corresponding 3D EPI TOPUP (Figure 11C), and 3D esEPI-BUDA (Figure 11D). 3D esEPI-BUDA and 3D fully-sampled EPI produced similar BOLD signal changes (~3%), which was higher than 3D EPI TOPUP images (~2%). The decrease of signal change for 3D EPI TOPUP images may be caused by the image interpolation in the TOPUP tool of FSL. The black bars represent the time period for the visual stimulus, which was 24 s, according to one example implementation;

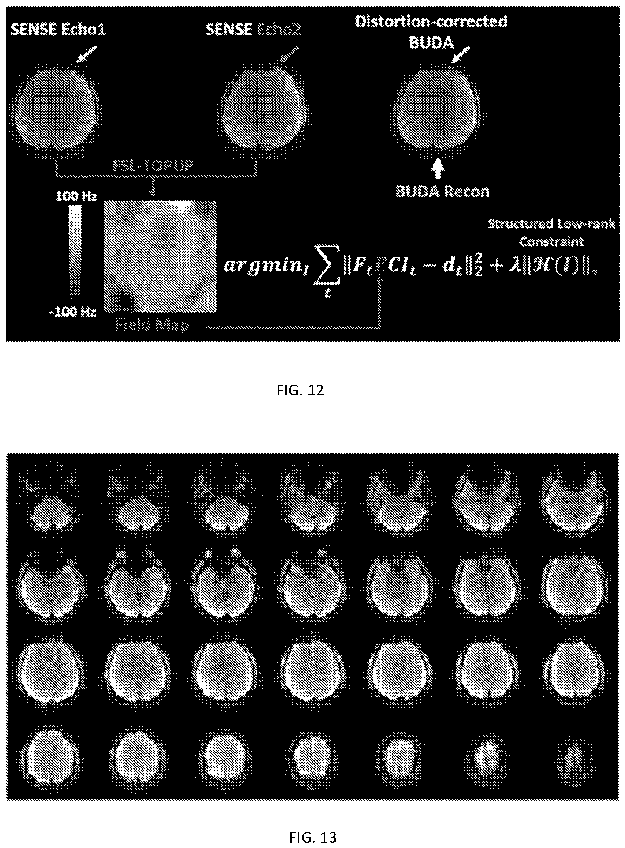

Figure 12 depicts the image reconstruction steps involved in BUDA using the human brain as an example. A field map estimated from separate blip-up and blip-down reconstructions is incorporated into a parallel imaging model to jointly reconstruct two datasets with low-rank regularization for geometric distortion correction. Quantitative measurement of SNR in a homogeneous region of white matter revealed an approximately 13.5% increase in the 3D BUDA image (SNR = 14.3) compared with the corresponding 3D SENSE images (SNR = 12.6), according to one example implementation;

Figure 13 depicts a set of representative whole-brain 3D images obtained in one volume TR using 3D esEPI-BUDA. Minimal geometric distortions are seen in the brain areas typically susceptible to distortion, according to one example implementation;

Figures 14A-D each depict a representative image of a slice selected from the 3D datasets of human brain acquired using 3D fully-sampled EPI with separately acquired blip-up (Figure 14A) and blip-down (Figure 14B) datasets, 3D esEPI-BUDA (Figure 14C), and 3D T1-weighted GRE (Figure 14D). As shown, 3D esEPI-BUDA (Figure 14C) produced images with considerably reduced geometric distortion (e.g., in the frontal lobe), when compared to the 3D fully-sampled EPI results with separately acquired blip-up and blip-down datasets (Figures 14A-14B). In all comparisons, the 3D T1-weighted GRE image (Figure14D) was used as a reference. The horizontal lines serve as a fiducial mark. Note that the image brightness is intentionally exaggerated to facilitate the comparisons, according to one example implementation;

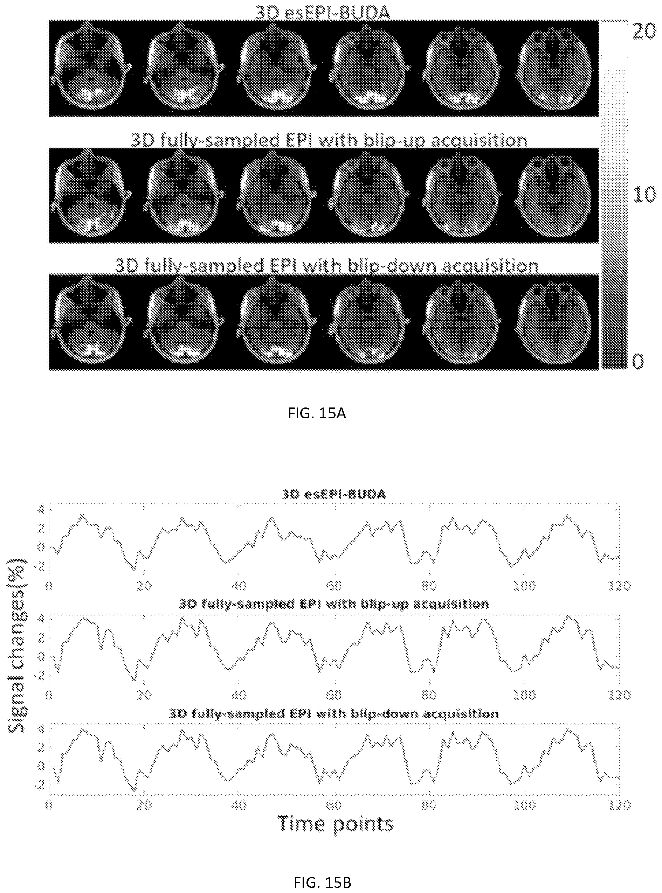

Figure 15A depicts visual fMRI activation maps of 3D esEPI-BUDA (first row), compared with 3D fully-sampled EPI with blip-up (second row) and blip-down (third row) acquisitions, according to one example implementation; and

Figures 15B S% depicts the corresponding time courses over 4 min and 48 s for the fMRI activation maps of Figure 15A that illustrate comparable BOLD signal changes of approximately 4.0%, according to one example implementation.

The drawings are for the purpose of illustrating examples, but it is understood that the disclosure is not limited to the arrangements and instrumentalities shown in the drawings.

## DETAILED DESCRIPTION

### I. Overview

The disclosed exemplary methods - esEPI-BUDA - advantageously integrate the blip-up and blip-down acquisitions into a single repetition time ("TR") or shot. Two interleaved k-space datasets with reversed k-space filling trajectories are acquired conjointly in one shot, followed by BUDA reconstruction to produce a distortion-corrected image. The esEPI-BUDA technique, method, systems, and computer-readable mediums are demonstrated in phantoms and the healthy human brain for functional activity mapping.

The disclosed systems, methods, and computer readable mediums can beneficially integrate blip-up and blip-down acquisitions in one shot, avoid shot-to-shot phase variations, eliminate inter-shot motion sensitivity, and reduce the scan times. These features can benefit clinical and research use of MRI on human subjects, as well as animals.

### II. Example Architecture

Figure 1 is a block diagram showing an operating environment 100 that includes or involves, for example, an MRI system 105 described below. Method 300 shown in Figure 3 and described below is an example of a method that can be implemented within this operating environment 100.

Figure 2 is a block diagram illustrating an example of a computing device 200, according to an example implementation, that is configured to interface with operating environment 100, either directly or indirectly. The computing device 200 may be used to perform functions of the method shown in Figure 3 and described below. In particular, computing device 200 can be configured to perform one or more functions, including integrating blip-up and blip-down acquisitions into a single shot and utilizing BUDA reconstruction to produce a distortion-corrected image, for example. The computing device 200 has a processor(s) 202, and also a communication interface 204, data storage 206, an output interface 208, and a display 210 each connected to a communication bus 212. The computing device 200 may also include hardware to enable communication within the computing device 200 and between the computing device 200 and other devices (e.g., not shown). The hardware may include transmitters, receivers, and antennas, for example.

The communication interface 204 may be a wireless interface and/or one or more wired interfaces that allow for both short-range communication and long-range communication to one or more networks 214 or to one or more remote computing devices 216 (e.g., a tablet 216a, a personal computer 216b, a laptop computer 216c and a mobile computing device 216d, for example). Such wireless interfaces may provide for communication under one or more wireless communication protocols, such as Bluetooth, Wi-Fi (e.g., an institute of electrical and electronic engineers (IEEE) 802.11 protocol), Long-Term Evolution (LTE), cellular communications, near-field communication (NFC), and/or other wireless communication protocols. Such wired interfaces may include Ethernet interface, a Universal Serial Bus (USB) interface, or similar interface to communicate via a wire, a twisted pair of wires, a coaxial cable, an optical link, a fiber-optic link, or other physical connection to a wired network. Thus, the communication interface 204 may be configured to receive input data from one or more devices and may also be configured to send output data to other devices.

The communication interface 204 may also include a user-input device, such as a keyboard, a keypad, a touch screen, a touch pad, a computer mouse, a track ball and/or other similar devices, for example.

The data storage 206 may include or take the form of one or more computer-readable storage media that can be read or accessed by the processor(s) 202. The computer-readable storage media can include volatile and/or non-volatile storage components, such as optical, magnetic, organic or other memory or disc storage, which can be integrated in whole or in part with the processor(s) 202. The data storage 206 is considered non-transitory computer readable media. In some examples, the data storage 206 can be implemented using a single physical device (e.g., one optical, magnetic, organic or other memory or disc storage unit), while in other examples, the data storage 206 can be implemented using two or more physical devices.

The data storage 206 thus is a non-transitory computer readable storage medium, and executable instructions 218 are stored thereon. The instructions 218 include computer executable code. When the instructions 218 are executed by the processor(s) 202, the processor(s) 202 are caused to perform functions.

The processor(s) 202 may be a general-purpose processor or a special purpose processor (e.g., digital signal processors, application specific integrated circuits, etc.). The processor(s) 202 may receive inputs from the communication interface 204 and process the inputs to generate outputs that are stored in the data storage 206 and output to the display 210. The processor(s) 202 can be configured to execute the executable instructions 218 (e.g., computer-readable program instructions) that are stored in the data storage 206 and are executable to provide the functionality of the computing device 200 described herein.

The output interface 208 outputs information to the display 210 or to other components as well. Thus, the output interface 208 may be similar to the communication interface 204 and can be a wireless interface (e.g., transmitter) or a wired interface as well. The output interface 208 may send commands to one or more controllable devices, for example.

The computing device 200 shown in Figure 2 may also be representative of a local computing device 200a in operating environment 100, for example, in communication with and electrically coupled to the MRI system 105. This local computing device 200a may perform one or more of the steps of the method 300 described below, may receive input from a user and/or may send image data and user input to computing device 200 to perform all or some of the steps of method 300.

Figure 3 shows a flowchart of example method 300 for an MRI system to generate an echo-shifted echo-planar imaging with blip up/down acquisition ("esEPI-BUDA") pulse sequence with RF pulses in a single shot and to acquire two k-space datasets within the single shot and to correct image distortion, according to an example implementation. Method 300 is an example method that could be used with the computing device 200 of Figure 2, for example. In some instances, components of MRI system 105 may be configured to perform the functions such that the components are configured and structured with hardware and/or software to enable such performance. Components of the devices and/or systems may be arranged to be adapted to, capable of, or suited for performing the functions, such as when operated in a specific manner. Method 300 may include one or more operations, functions, or actions as illustrated by one or more of blocks 305-315. Although the blocks are illustrated in a sequential order, some of these blocks may also be performed in parallel, and/or in a different order than those described herein. Also, the various blocks may be combined into fewer blocks, divided into additional blocks, and/or removed based upon the desired implementation.

It should be understood that for this and other processes and methods disclosed herein, flowcharts show functionality and operation of one possible implementation of the present examples. In this regard, each block may represent a module, a segment, or a portion of program code, which includes one or more instructions executable by a processor for implementing specific logical functions or steps in the process. The program code may be stored on any type of computer readable medium or data storage, for example, such as a storage device including a disk or hard drive. Further, the program code can be encoded on a computer-readable storage media in a machine-readable format, or on other non-transitory media or articles of manufacture. The computer readable medium may include non-transitory computer readable medium or memory, for example, such as computer-readable media that stores data for short periods of time such as register memory, processor cache and Random Access Memory (RAM). The computer readable medium may also include non-transitory media, such as secondary or persistent long-term storage, like read only memory (ROM), optical or magnetic disks, compact-disc read only memory (CD-ROM), for example. The computer readable media may also be any other volatile or non-volatile storage systems. The computer readable medium may be considered a tangible computer readable storage medium, for example.

In addition, each block in Figure 3, and within other processes and methods disclosed herein, may represent circuitry that is wired to perform the specific logical functions in the process. Alternative implementations are included within the scope of the examples of the present disclosure in which functions may be executed out of order from that shown or discussed, including substantially concurrent or in reverse order, depending on the functionality involved, as would be understood by those reasonably skilled in the art.

## III. Example Methods

The following method 300 may include one or more operations, functions, or actions as illustrated by one or more of blocks 305-315. Although the blocks are illustrated in a sequential order, these blocks may also be performed in parallel, and/or in a different order than those described herein. Also, the various blocks may be combined into fewer blocks, divided into additional blocks, and/or removed based upon the desired implementation. Alternative implementations are included within the scope of the examples of the present disclosure in which functions may be executed out of order from that shown or discussed, including substantially concurrent or in reverse order, depending on the functionality involved, as would be understood by those reasonably skilled in the art.

Referring now to Figure 3, Figure 3 shows a flowchart of an example method 300 for using the MRI system 105 electrically coupled to a computing device 200, according to an example implementation. As used herein, "electrically coupled" refers to coupling using a conductor, such as a wire or a conductible trace, as well as inductive, magnetic, and wireless couplings. The computing device 200 may be local or remote to the MRI system 105.

Method 300 includes, at block 305, the MRI system 105 generating an echo-shifted echo-planar imaging with blip up/down acquisition ("esEPI-BUDA") pulse sequence. The esEPI-BUDA pulse sequence includes a first radiofrequency ("RF") pulse and a second RF pulse, the first RF pulse followed by a first echo-train that is interleaved with the first and the second RF pulses, and the second RF pulse followed by a second echo-train such that the first and the second echo-trains have opposite phase-encoding blip gradient polarities to traverse echo planar imaging ("EPI") k-space in a reversed order. Then, at block 310, in response to the pulse sequence being generated, the MRI system 105 acquires two k-space datasets within a single shot (i.e., repetition time, or TR). Next, at block 315, the MRI system 105 corrects image distortion based on the two acquired k-space datasets.

In one optional implementation, the two k-space datasets acquired in a single shot (or TR) may be configured to eliminate or substantially reduce inter-shot phase errors caused by MRI system imperfection, subject motion, and/or other factors. In various example implementations, correcting image distortion may conducted through joint reconstruction or separately followed by geometric corrections using an algorithm, such as EPI TOPUP. In a further optional implementation, the computing device 200 is an MRI reconstruction processor of the MRI system 105 that conducts joint image reconstruction.

In one optional implementation, the first RF pulse has a flip angle of $\alpha$ and the second RF pulse has a flip angle of $\beta$, with $\alpha$ and $\beta$ satisfying the following condition:

$$sin\,\alpha \cdot cos^2(\beta/2) = \, cos\alpha \cdot sin\beta.$$

In one optional implementation, generating the esEPI-BUDA pulse sequence further includes the MRI system 105 generating a plurality of echo-shifting gradients applied along a direction perpendicular to the imaging plane. In a further optional implementation, generating the plurality of echo-shifting gradients includes the MRI system 105 generating a first echo-shifting gradient with an area of *G'* and thereby dephasing transverse magnetization from the first RF pulse, where A is the absolute value of the slice-refocusing gradient area (or one half of the slice-selection gradient area as shown in Figure 4A) and *G* is the absolute value of the nominal dephasing/rephasing gradient area as detailed thereafter. After the transverse magnetization is dephased, the MRI system generates the second RF pulse and thereby excites the stored longitudinal magnetization. After the second RF pulse is generated, the MRI system 105 generates the second echo-shifting gradient with an area of *-G* and thereby dephases transverse magnetization from the second RF pulse and rephases a signal produced by the first RF pulse. And after the signal produced by the first RF pulse is acquired, generating, via the MRI system, the third echo-shifting gradient with an area of *G* and thereby dephasing transverse magnetization from the first RF pulse and rephasing a signal produced by the second RF pulse. In a further optional implementation, *G' = G - A* with *A* being the absolute value of the area of a slice-refocusing gradient associate with the first or the second RF pulse.

In one optional implementation the first echo-train with blip-up phase-encoding acquires the rephased signal produced by the first RF pulse.

In yet another optional implementation, generating the plurality of echo-shifting gradients further includes, after the first echo-train is generated, the MRI system generating a third echo-shifting gradient with an area of *G* and thereby rephasing a signal produced by the second RF pulse and dephasing the signal produced by the first RF pulse.

In one optional implementation, the second echo-train with blip-down phase-encoding acquires the rephased signal produced by the second RF pulse.

In one optional implementation, the esEPI-BUDA pulse sequence further includes, after acquiring the rephased signal produced by the first RF pulse and before acquiring the rephased signal produced by the second RF pulse, the MRI system 105 generating a gradient having one half (½) of an individual phase-encoding blip gradient area ($G_y$).

In one optional implementation, the first and second RF pulses and their associated first and second EPI echo-trains and echo-shifting gradients are extended to a plural number greater than two.

In another optional implementation, the esEPI-BUDA pulse sequence is applied for two-dimensional or three-dimensional functional MRI, diffusion MRI, perfusion MRI, and/or other MRI applications where echo-train-based echo planar imaging acquisitions are employed.

In one optional implementation, the MRI system under-samples k-space data from the first echo-train and the second echo-train and thereby shortens a length of each of the first echo-train and the second echo-train.

In one optional implementation, conducting joint image reconstruction based on the two acquired k-space datasets further includes the MRI system 105 generating dynamic maps of a main magnetic field (i.e., $B_0$ field). And the MRI system incorporating the dynamic maps of a main magnetic field into a forward joint parallel imaging reconstruction model with Hankel structured low-rank constraints and thereby correcting the image geometric distortion.

In another optional implementation, conducting joint image reconstruction based on the two acquired k-space datasets further includes the MRI system 105 combining the two acquired k-space datasets and thereby improving the image signal-to-noise ratio.

### IV. Non-Transitory Computer-Readable Mediums

As discussed above, a non-transitory computer-readable medium having stored thereon program instructions that upon execution by an MRI system 105 electrically coupled to a computing device 200 may be utilized to cause performance of any of functions of the foregoing methods.

As one example, a non-transitory computer-readable medium having stored thereon program instructions that upon execution by a processor, cause performance of a set of steps includes an MRI system 105 generating an echo-shifted echo-planar imaging with blip up/down acquisition ("esEPI-BUDA") pulse sequence. The esEPI-BUDA pulse sequence includes a first radiofrequency ("RF") pulse and a second RF pulse, the first RF pulse followed by a first echo-train that is interleaved with the first and the second RF pulses, and the second RF pulse followed by a second echo-train such that the first and the second echo-trains have opposite phase-encoding blip gradient polarities to traverse echo planar imaging ("EPI") k-space in a reversed order. In response to the pulse sequence being generated, the MRI system 105 acquires two k-space datasets within a single shot. The MRI system 105 then corrects image distortion based on the two acquired k-space datasets. In various example implementations, correcting image distortion may be conducted through joint reconstruction or separately followed by geometric corrections using an algorithm, such as EPI TOPUP.

In one optional implementation, the MRI system 105 generating the esEPI-BUDA pulse sequence further includes the MRI system 105 generating a plurality of echo-shifting gradients applied along a direction perpendicular to the imaging plane.

In another optional implementation, the MRI system 105 generating the plurality of echo-shifting gradients includes the MRI system 105 generating a first echo-shifting gradient with an area of ($G$ - $A$) and thereby dephasing transverse magnetization from the first RF pulse, where A is the absolute value of the slice-refocusing gradient area (or one half of the slice-selection gradient area as shown in Figure 4A) and $G$ is the absolute value of the nominal dephasing/rephasing gradient area as detailed thereafter. After the transverse magnetization is dephased, the MRI system 105 generates the second RF pulse and thereby excites the stored longitudinal magnetization. After the second RF pulse is generated, the MRI system 105 generates the second echo-shifting gradient with an area of -$G$ and thereby dephases transverse magnetization from the second RF pulse and rephases a signal produced by the first RF pulse. And after the signal produced by the first RF pulse is acquired, the MRI system generates the third echo-shifting gradient with an area of $G$ and thereby dephases transverse magnetization from the first RF pulse and rephases a signal produced by the second RF pulse. In a further optional implementation, $G' = G - A$ with $A$ being the absolute value of the area of a slice-refocusing gradient associate with the first or the second RF pulse.

In one optional implementation, the non-transitory computer-readable medium includes the first echo-train with blip-up phase-encoding acquiring the rephased signal produced by the first RF pulse.

In another optional implementation, generating the plurality of echo-shifting gradients further includes, after the first echo-train is generated, the MRI system 105 generating a third echo-shifting gradient with an area of $G$ and thereby rephasing a signal produced by the second RF pulse and dephasing the signal produced by the first RF pulse.

In a further optional implementation, the non-transitory computer-readable medium includes the second echo-train with blip-down phase-encoding acquiring the rephased signal produced by the second RF pulse.

In still another optional implementation, the esEPI-BUDA pulse sequence includes, after acquiring the rephased signal produced by the first RF pulse and before acquiring the rephased signal produced by the second RF pulse, the MRI system 105 generating a gradient having one half (½) of an individual phase-encoding blip gradient area ($G_y$).

In one optional implementation, the MRI system 105 conducting joint image reconstruction based on the two acquired k-space datasets includes the MRI system generating dynamic maps of a main magnetic field. Then the MRI system 105 incorporates the dynamic maps of a main magnetic field into a forward joint parallel imaging reconstruction model with Hankel structured low-rank constraints and thereby corrects the image geometric distortion.

In one optional implementation, the MRI system 105 conducting joint image reconstruction based on the two acquired k-space datasets includes the MRI system 105 combining the two acquired k-space datasets and thereby improving the image signal-to-noise ratio.

## V. Example 1

### Materials and methods:

*3D esEPI-BUDA sequence:*

Figures 4A-B show an example of the 3D esEPI-BUDA pulse sequence and its corresponding k-space trajectories. 3D esEPI-BUDA uses two RF pulses to acquire the blip-up and blip-down datasets in a single TR (or shot). The two signals from the two RF pulses are time-shifted and individually selected by three dephasing/rephasing gradients (or echo-shifting gradients; ($G$ - $A$), -$G$, and $G$) applied along the slab-selection direction ($G_z$). The first echo-shifting gradient with an area of ($G$ - $A$) acts as a spoiler to dephase the transverse magnetization shortly after the first RF pulse $\alpha$. (Note that A is the net area of slab-refocusing gradient associated with the first RF pulse.) After the transverse magnetization is dephased, the second RF pulse $\beta$ is applied to excite the stored longitudinal magnetization, followed by the second echo-shifting gradient with an area of -$G$. This gradient dephases the transverse magnetization from RF pulse $\beta$, while, together with the net slab-selection gradient area ($2A - A = A$) associated with RF pulse $\beta$, rephasing the transverse magnetization produced by RF pulse $\alpha$.

The rephased signal is acquired by the first EPI echo-train with blip-up phase-encoding (black). The third and final echo-shifting gradient with an area of $G$ is placed after the first readout echo-train to rephase the signal produced by RF pulse $\beta$, while dephasing the remaining transverse magnetization from RF pulse $\alpha$. The rephased signal is acquired by the second echo-train with blip-down phase-encoding (gray). k-Space data from both echo-trains are under-sampled (e.g., by two-fold) to shorten the echo-train length, thus enabling short and consistent TEs (e.g., 30 ms for fMRI at 3T) in both acquisitions. To make the echo times of the two echo-trains the same, the time delay between the two RF pulses ($\alpha$ and $\beta$) is determined by the center-to-center length of the two echo-trains (i.e., the summation of the third echo-shifting gradient width plus the duration of an individual echo-train).

The signal in the first echo-train is attenuated by $cos^2(\beta/2)$, and thus is proportional to $M_0 \cdot sin\,\alpha \cdot cos^2(\beta/2)$, where the $M_0$ is the longitudinal magnetization. The signal in the second echo-train is proportional to $M_0 \cdot cos\,\alpha \cdot sin\,\beta$, provided that the time delay (~20 ms) between the two RF pulses is short as compared to the T1 value of the tissues. To equalize the signals for the two echo-trains, the flip angles $\alpha$ and $\beta$ need to satisfy the following condition:

$$sin\,\alpha \cdot cos^2(\beta/2) = cos\alpha \cdot sin\beta$$

The esEPI-BUDA sequence also contains a small gradient with ½ phase-encoding blip area ($G_y$) prior to the second echo-train so that the two k-space trajectories can be interleaved (Figure 4B). This allows the two k-space datasets to be effectively used in the Hankel structured low-rank image reconstruction. In principle, the esEPI-BUDA pulse sequence described above can be used for 2D or 3D imaging. In the former case, the slab-selection gradient along the $G_z$-axis is reduced to a slice-selection gradient, while in the latter case, a conventional stepping phase gradient is applied along the $G_z$-axis, providing spatial localization for both echo trains as shown in Figure 4A.

*Imaging Experiments:*

For fMRI applications, a 3D version of esEPI-BUDA was implemented on a GE MR750 3T scanner (GE Healthcare, Waukesha, Wisconsin, USA) to avoid the excessive SNR penalty that would incur in 2D implementations. Phantom and human *in vivo* experiments were performed using a 32-channel head coil (Nova Medical, Inc., Wilmington, Massachusetts, USA) to demonstrate the proposed esEPI-BUDA technique.

In the phantom experiment, a GE DQA (Daily Quality Assurance) phantom was used to validate the pulse sequence and its associated 3D image reconstruction. Phantom images from the 3D esEPI-BUDA sequence were acquired with the following parameters: TR/TE = 100/40 ms, volume TR ($TR_{vol}$) = 3.2 s (where the $TR_{vol}$ is defined as the time taken to acquire a 3D volume), flip angles: $\alpha \approx \beta = 15°$, FOV = 180 × 180 × 128 $mm^3$, acquisition matrix = 72 × 72 × 32, spatial resolution = 2.5 × 2.5 × 4.0 $mm^3$, under-sampling factor along the in-slab phase-encoding direction = 2 (i.e., the length of each echo-train ("ETL") = 36; total length of two echo-trains = 72), and echo spacing = 0.528 ms. The corresponding k-space data from the two echo-trains were separately reconstructed using SENSE, followed by joint image reconstruction (see *Image reconstruction*). For comparison, images over the same volume were also acquired using 3D fully-sampled EPI (flip angle = 15°) with blip-up and blip-down acquisitions separately. To establish a reference to assess the improvement in image distortion reduction, additional images were obtained using a conventional 3D fast SPGR sequence (TRITE = 10.6/1.4 ms, flip angle = 10°, FOV = 180 × 180 × 128 $mm^3$, and acquisition matrix = 72 × 72 × 32).

The *in vivo* experiment aimed at demonstrating the 3D esEPI-BUDA sequence for fMRI with visual stimulation. The imaging parameters were: TR/TE = 75/30 ms, $TR_{vol}$ = 2.4 s, $\alpha \approx \beta$ = 15°, FOV = 220 × 220 × 128 $mm^3$, acquisition matrix = 72 × 72 × 32, spatial resolution = 3.1 × 3.1 × 4.0 $mm^3$, under-sampling factor along the in-slab phase-encoding direction = 2, and echo spacing = 0.456 ms. For comparison, images over the same volume were also acquired using 3D fully-sampled EPI (flip angle = 15°) with blip-up and blip-down acquisitions separately. Similar to the phantom experiment, a conventional 3D fast SPGR image with matched FOV was acquired as a reference (TR/TE = 10.5/1.4 ms, flip angle = 10°, FOV = 220 × 220 × 128 $mm^3$, and acquisition matrix = 72 × 72 × 32).

For the *in vivo* experiment, visual stimulation was delivered using a commercial system (SensaVue, Invivo Corporation, Gainesville, Florida, USA) with a dark-gray and light-gray checkboard pattern flashing at 8 Hz. This block-design paradigm contained six 48 s blocks, each with a 24 s stimulation period and a 24 s rest. The total acquisition time was 4 min and 48 sec. Six healthy human subjects (33.2 ± 5.9 years) were asked to fixate on the cross-hair presented at the center of the visual field during the experiment. The fMRI stimulation delivery system was integrated with an infrared camera focusing on the subject's pupil so that the subject's motion and attentiveness to the fMRI task were monitored in real time. The camera was positioned at a 90° angle with respect to the optical pathway of the visual stimulation light. A large hot mirror (35" x 17") was positioned at a 45° angle with respect to both the incident infrared and visible light beams, allowing 98% transmission of visible light while reflecting 97% of infrared.

*Image reconstruction:*

The raw k-space data acquired with the 3D esEPI-BUDA sequence were preprocessed before image reconstruction: First, phase correction was performed to remove the Nyquist ghosting artifacts. The zeroth- or first-order phase differences between the odd and even encodings of each echo-train were corrected based on a reference scan by setting the amplitude of the phase-encoding gradients to zero. Second, to speed up the image reconstruction, a model based on the principal component analysis ("PCA") was used to linearly concatenate the raw data from 32 channels into 16 channels. Coil sensitivity profile was estimated using the ESPIRiT approach with the data from the FOV-matched distortion-free 3D SPGR sequence. After the preprocessing, distortion-corrected images were reconstructed from the k-space data with the pipeline described in the following paragraphs.

Figure 5 shows the steps involved in 3D esEPI-BUDA image reconstruction. Each under-sampled echo-train dataset (i.e., blip-up and blip-down) first underwent 3D SENSE reconstruction individually using the following equation:

$$\tilde{I} = \underset{I}{argmin} \|UF(SI) - d\|_2^2$$

where $U$ is the sampling mask of k-space locations, $F$ represents the Fourier transform operator, $S$ is the coil sensitivity, $d$ is the k-space dataset, $I$ is the 3D SENSE-reconstructed image, and $\tilde{I}$ is the resultant $I$ after iterations. A 3D projection onto convex sets ("POCS") algorithm was employed to solve the above equation.

After obtaining the two 3D SENSE images with blip-up and blip-down encoding individually, TOPUP in FSL was used to estimate a $B_0$-field map $E$, which was subsequently incorporated to jointly reconstruct the data from both echo-trains, as follows:

$$\tilde{I} = argmin_I \sum_{t=1}^{2} \|U_t F_t(ESI_t) - d_t\|_2^2 + \lambda \|\mathcal{H}(I)\|_*$$

where $t$ is the echo-train index (1 or 2), $U_t$ is the sampling mask of $t^{th}$ echo-train, $F_t$ is the fast Fourier transform operator for $t^{th}$ under-sampled echo-train, E is the $B_0$-field map estimated using TOPUP in FSL, as shown in Figure 5, $S$ is the coil sensitivity information, $I_t$ is the target distortion-corrected image, and $d_t$ is the acquired under-sampled k-space data of $t^{th}$ echo-train. $\mathcal{H}(I)$ represents the Hankel low-rank matrix which enforces low-rankness among different echo-trains, $\|.\|_*$ denotes the nuclear norm of the matrix, which is the sum of singular values, and $\lambda$ is the parameter to tune the weight of structured low-rank regularization.

In the 3D esEPI-BUDA joint image reconstruction, the Hankel structured low-rank constraint mitigated the phase errors between the two echo-trains and background noise based on the hypothesis that the k-space data of MR images typically have limited spatial support and/or slowly varying phase. Herein, the Hankel matrix was constructed by consecutively picking up 9 × 9 × 9 neighborhood points in k-space from each echo-train as a Hankel-block, followed by concatenating them in the column dimension. A POCS-like approach was used to solve this equation. In the POCS-iterative framework, root-mean-square error of less than 1% between two successive iterations was chosen to indicate convergency.

All image reconstructions were implemented in MATLAB (R2019b; the Mathworks, Natick, MA, USA) for off-line reconstruction on a Linux server (CentOS, Intel (R) Core (TM) i9-7920X CPU @ 2.90 GHz and 128 GB RAM).

*Data analysis:*

To demonstrate the performance of 3D esEPI-BUDA in terms of data acquisition efficiency and distortion correction effectiveness, images from the DQA phantom and fMRI experiments were evaluated and compared between 3D esEPI-BUDA and a conventional approach with separate blip-up and blip-down acquisitions followed by a correction method using TOPUP in FSL. This method (called 3D EPI TOPUP thereafter) performs image-domain registration between the two separately acquired datasets, each with 3D full sampling. In the DQA phantom experiment, the horizontal low-signal block (as indicated by the arrow in image G of Figure 6) serves as a fiducial mark to qualitatively assess the effectiveness of geometric distortion correction produced by the 3D esEPI-BUDA with joint reconstruction and the two separate blip-up/down acquisitions with 3D EPI TOPUP, respectively. In the comparison, an image acquired by the 3D SPGR sequence was used as a reference. Three quantitative image analyses were also performed, including structural similarity (SSIM), normalized root-mean-square error (NRMSE), and SNR to compare the image quality.

In the process of image reconstruction of fMRI data, $B_0$-field maps ($E$) were estimated at each time point (i.e., $TR_{vol}$) as shown in the foregoing equation. The mean value and the standard deviation of $E$ across the time points were calculated to evaluate the time evolution of $B_0$-field maps. The dynamic change of the $B_0$-field map was analyzed at the frontal and occipital lobes. The resulting real-time spatial dislocation could be calculated by:

$$\Delta d = \frac{2\pi\Delta f}{BW \cdot \Delta k}$$

where $\Delta f$ is the amount of off-resonance in Hertz that can be derived from the $B_0$-field map, $\Delta k$ is the k-space sampling interval, and $BW$ is the sampling bandwidth along the blipped phase-encoding direction, which is inversely related to the echo spacing. $\Delta d$ was used to quantify the geometric distortion at each point in the fMRI experiment.

fMRI data were analyzed using SPM8 on MATLAB. Motion correction and spatial smoothing (FWHM = 6 mm) were applied to magnitude images, followed by statistical analyses using a general linear model for activation detection with a *P*-value threshold (FWE corrected) of < 0.05 and a spatial cluster size of at least 30 pixels. The MarsBar toolbox was used to extract and analyze the time courses. Averaged time courses with standard deviations across the subjects were compared among 3D esEPI-BUDA, the two separately acquired datasets with blip-up and blip-down prior to distortion correction, and distortion-corrected images using 3D EPI TOPUP.

**Results:**

Figure 6 displays images from the phantom experiment, including 3D fully-sampled EPI with separate blip-up (image A) and blip-down (image B) acquisitions, the corresponding 3D EPI TOPUP (image C), individually SENSE-reconstructed images from the first (image D) and second (image E) echo train of the 3D esEPI-BUDA sequence, a resultant esEPI-BUDA image (F), and a conventional 3D SPRG (image G) as a reference. For each display, a representative image corresponding to slice No. 16 of the DQA phantom was selected from the 3D volumetric dataset. The horizontal black block in the middle of the phantom (see the arrow in image (G) of Figure 6) was bent upwards or downwards in (A), (B), (D), and (E). Comparison of the first-row images in Figure 6 indicates that the sEPI-BUDA sequence produced high quality images (D) and € despite a two-fold scan time reduction as compared with the separate acquisition strategy for images (A) and (B). Images (D) and (E) exhibited less distortion (see the central block in the phantom) because of the use of parallel imaging. The second row of images show that both 3D EPI TOPUP in FSL and the joint k-space reconstruction employed in 3D esEPI-BUDA effectively corrected the image distortion with similar performance. Using the distortion-free SPGR image in image (G) of Figure 6 as a reference, the esEPI-BUDA image (F) of Figure 6 exhibited a higher degree of similarity with an SSIM of 0.91 and an NRMSE of 0.06 than the image reconstructed from two separate acquisitions (image (C) of Figure 6; SSIM = 0.87; NRMSE = 0.08). Quantitative measurement of SNR in the phantom revealed an approximately 42.2% increase in the 3D esEPI-BUDA image (image F of Figure 6; SNR = 86.4) compared with the individual 3D SENSE-reconstructed images (images (D) and (E) of Figure 6; SNR = 60.8). However, the SNR decreased by approximately 9.3% and 6.0% compared with the 3D fully-sampled EPI images (images (A) and (B) of Figure 6; SNR = 95.3) and the resultant EPI TOPUP image (image (C) of Figure 6; SNR = 91.9), respectively.

The performance of the distortion correction on a representative human subject (26-year-old male) is demonstrated in Figures 8A-C. Compared with the SENSE-reconstructed EPI images using the two individual echo trains (Figures 8A and 8B), esEPI-BUDA reduced the degree of image distortion and increased the SNR (Figure 8C). This becomes more apparent by analyzing a representative section selected from the 3D volume (Figure 9). Similar to the phantom results in Figure 6, each echo train in esEPI-BUDA produced good image quality as compared to the images acquired separately with the doubled scan time (first row of images in Figure 9). Compared to the images (A) and (B) in Figure 9, the reduced image distortion (e.g., in the frontal area) in images (D) and (E) in Figure 9 was attributed to the use of parallel imaging in esEPI-BUDA in an attempt to shorten the individual echo-train length. Both 3D EPI TOPUP on the individually acquired images and joint reconstruction employed in esEPI-BUDA effectively corrected the dilation and compression artifacts of the images (C) and (F) of Figure 9, when a conventional SPGR image was used as a reference (image (G) of Figure 9).

Figure 7A shows the dynamic $B_0$ field maps in the fMRI experiment obtained using 3D esEPI-BUDA. The time evolution of the $B_0$ field maps was captured with a temporal resolution of 2.4 s (i.e., volume TR). Each $B_0$ field map corresponded to a specific time point in the fMRI scan (numbered by 1, 2, ..., 120), and the collection of the images spanned a duration of 4 min and 48 sec. Representative $B_0$ field evolutions obtained from the frontal and occipital lobes are displayed in Figure 7B. The $B_0$-field values (mean $\pm$ standard deviation) in the two areas were -17.1 $\pm$ 1.2 Hz and 5.9 $\pm$ 1.0 Hz, respectively. During the entire time course, the maximal $B_0$-field difference (maximum value - minimum value) was approximately 6.2 Hz and 4.7 Hz for the frontal and occipital lobes, respectively. The resulting variations of spatial dislocation $\Delta d$ were estimated to be 1.95 mm and 1.47 mm (Figure 7C), respectively. These were approximately one half of the voxel size, indicating the necessity of obtaining the $B_0$-field map at each time point to dynamically correct the geometric distortion in an fMRI scan.

Results from the visual fMRI experiments are illustrated in Figure 10, where five contiguous activation maps are overlaid on the corresponding T1-weighted images. fMRI activations were observed in the visual cortex, as expected. The activation maps of 3D esEPI-BUDA co-registered better with the T1-weighted structure images than the two separately acquired 3D EPI images and their resultant 3D EPI TOPUP images in which the activations were beyond the border of brain parenchyma. This illustrates the advantage of esEPI-BUDA, which is capable of dynamically correcting the distortions during the fMRI time course, over the conventional approach. Comparable average activated volumes across subjects were detected (separate blip-up acquisition: 30.6 $cm^3$, separate blip-down acquisition: 25.3 $cm^3$, 3D EPI TOPUP: 30.7 $cm^3$, and 3D esEPI-BUDA: 27.2 $cm^3$). The average time courses across the six subjects at the visual cortex are shown in Figures 11A-D, where the 3D esEPI-BUDA sequence and the sequences with separate acquisitions produced similar BOLD signal changes (~3%), which was higher than 3D EPI TOPUP images (~2%). The decreased signal change in 3D EPI TOPUP was likely caused by the data interpolation in the TOPUP tool of FSL. The consistency in both the average activated volume and the BOLD signal change indicates the potential of using the 3D esEPI-BUDA sequence for BOLD activation detection.

As set forth herein, an exemplary esEPI-BUDA technique has been demonstrated, in which two interleaved k-space datasets with reversed k-space trajectories can be acquired in a single shot. Compared to traditional BUDA techniques for image distortion correction, esEPI-BUDA offers a major advantage by halving the scan times. With simultaneous blip-up and blip-down acquisitions in a single shot, esEPI-BUDA can also be more resilient to motion and allow $B_0$-field maps to be estimated dynamically throughout a time series. The latter is particularly desirable as the $B_0$-field maps can be incorporated into the forward joint parallel imaging reconstruction model with Hankel structured low-rank constraint to correct the geometric distortion.

As an attractive technique for distortion correction in EPI, BUDA has been successfully applied to susceptibility-weighted imaging, diffusion-weighted imaging, and T2 mapping. Extension to fMRI has been challenging as the acquisitions of blip-up and blip-down data in two separated TRs lead to a substantially degraded temporal resolution (i.e., > 4 s). By utilizing the echo-shifting strategy, esEPI-BUDA incorporates two echo-trains into a single pulse sequence, overcoming the temporal resolution limitation as demonstrated by the short volume TR (e.g., 2.4 s) achieved in fMRI experiments.

Owing to the pair-wise acquisition of the blip-up and blip-down datasets, esEPI-BUDA is capable of capturing real-time $B_0$-field temporal variations in an fMRI scan. The temporal variations can be a reflection of subject motion, physiological respiration, heating of the gradient system, mechanic vibration, and/or other system instabilities. Although TOPUP in FSL is effective for correcting distortion at a single time-point (image (C) in both Figures 6 and 9), distortion correction based on retrospective techniques to estimate the $B_0$-field map cannot account for the dynamic off-resonance effects. In contrast, esEPI-BUDA is capable of monitoring the dynamic $B_0$-field change and using that information to correct image distortion at each time point (Figures 7A-C and 10). This can be particularly useful for fMRI, diffusion imaging, and arterial spin labeling whose scan times can last for several minutes or longer.

In parallel to the two-fold scan time reduction by using the echo-shifting strategy, conventional parallel imaging was also employed in the $G_y$-direction with an acceleration factor of two to shorten the echo-train length for the individual blip-up and blip-down acquisitions. The use of parallel imaging also reduced the image distortion (images (A) and (B) of Figure 9 vs. images (D) and (E) of Figure 9) as a result of an increased effective bandwidth along the blipped phase-encoding direction. Higher acceleration factors can be potentially achieved by applying data under-sampling in both $k_z$ and $k_y$ directions to reduce not only the echo-train length but also the volume TR. An example is to employ 2D CAIPIRINHA (controlled aliasing in parallel imaging results in higher acceleration) to accomplish multi-dimensional acceleration. The feasibility of using higher acceleration factors and temporal resolutions in esEPI-BUDA will be further explored in future studies.

In the esEPI-BUDA sequence, the blip-up and blip-down echo-trains were interleaved so that the complete k-space datasets could be used in the joint reconstruction. The joint reconstruction can significantly reduce the g-factor noise penalty when compared with reconstructions on the blip-up and blip-down acquisitions separately. In this study, Hankel structured low-rank regularization was also exploited to strengthen the joint of the blip-up and blip-down datasets by a low-rank constraint, which not only reduces the g-factor, but also removes the phase errors between different echo-trains without the need of navigation. The echo-shifting strategy results in a theoretical SNR loss of $(1 - cos^2(\beta/2))$. For the phantom studies, the theoretical SNR loss was only ~1.7% because of a low flip angle ($\beta$ = 15°). The experimentally measured SNR loss (9.3%), however, was higher, likely caused by the noise amplification (g-factor) during image reconstruction.

This example study has limitations. First, 3D fMRI was employed as an example to illustrate the esEPI-BUDA technique. Although the concept of esEPI-BUDA can be applied to both 2D and 3D acquisitions, esEPI-BUDA for 2D multi-slice fMRI would require a large flip angle of the RF excitation pulse. With a large flip angle, however, the SNR loss due to the use of the echo-shifting strategy can be substantial. For example, with optimal flip angles of $\alpha \simeq 47°$ and $\beta \simeq 56°$, the SNR in 2D esEPI-BUDA would be reduced by ~50% from that of a conventional 2D EPI sequence with a 90° RF excitation pulse for excitation. Performing 2D esEPI-BUDA acquisitions at a higher magnetic field (e.g., 7 Tesla) may compensate for such SNR loss. Second, the echo-shifting strategy limits the shortest TE achievable in esEPI-BUDA. Assuming that partial Fourier k-space encoding is not employed, the shortest TE is given by $0.5 \times T_{ss} + 2 \times T_{es} + 1.5 \times esp \times ETL$, where $T_{ss}$ is the duration of slab-selection gradient, $T_{es}$ is the duration of the echo-shifting gradient, $esp$ is the echo spacing in the echo-train, and *ETL* is the echo-train length of each echo-train. The analysis showed that the minimum TE of ~28 ms could be achieved in the visual fMRI experiment, which was fortunately adequate for BOLD contrast. A lengthy echo-train needed by high spatial resolution, however, can increase the minimum TE in the 3D esEPI-BUDA sequence. An excessively long TE reduces the SNR and increases the sensitivity to magnetic field inhomogeneities and flow effects. The issue can be mitigated by reducing the duration of the echo-shifting gradients and/or incorporating parallel imaging with a higher acceleration factor to shorten the echo-trains. The latter, however, can decrease the SNR, exacerbate residual aliasing, and compromise BOLD detectability.

As set forth herein, an exemplary technique, esEPI-BUDA, has been demonstrated to enable 3D distortion-corrected whole-brain 3D fMRI without increasing the scan time. The method integrates the blip-up and blip-down data acquisitions in a single shot, followed by joint reconstruction. The integrated data acquisition strategy also produces time resolved $B_0$-field maps that can be incorporated into image reconstruction to achieve dynamic image distortion correction. The method has been demonstrated on the phantom and human brain. Distortion-corrected 3D echo-planar images were successfully obtained with adequate SNR and BOLD sensitivity. With these demonstrations, esEPI-BUDA methods are expected to benefit other neuroimaging applications such as 3D/2D fMRI, diffusion imaging, and perfusion imaging.

**VI. Example 2**

**Methods:**

3D esEPI-BUDA:

Figures 4A-B show the principle of 3D esEPI-BUDA pulse sequence and its corresponding k-space trajectories. 3D esEPI-BUDA uses two RF pulses to acquire the blip-up and blip-down datasets in a single TR (or shot). The two signals from the two RF pulses are time-shifted and individually selected by three dephasing/rephasing gradients (or echo-shifting gradients; (*G - A*), *-G ,* and *G*) applied along the slab-selection direction ($G_z$). The first echo-shifting gradient with an area of (*G - A*) acts as a spoiler to dephase the transverse magnetization shortly after the first RF pulse $\alpha$. After the transverse magnetization is dephased, the second RF pulse $\beta$ is applied to excite the stored longitudinal magnetization, followed by the second echo-shifting gradient with an area of *-G.* This gradient dephases the transverse magnetization from RF pulse $\beta$, while rephasing the transverse magnetization produced by RF pulse $\alpha$. The rephased signal is acquired by the first EPI echo-train with blip-up phase-encoding (black). The final echo-shifting gradient with an area of *G* is placed after the first readout echo-train to rephase the signal produced by RF pulse $\beta$, while dephasing the signal from RF pulse $\alpha$. The rephased signal is acquired by the second echo-train with blip-down phase-encoding (gray). k-Space data from both echo-trains are under-sampled (e.g., by two-fold) to shorten the echo-train length, thus enabling short and consistent TEs (e.g., 30 ms for fMRI at 3T) for both acquisitions. To equalize the signals for the two echo-trains, the flip angles $\alpha$ and $\beta$ need to satisfy the following condition:

$$\sin\alpha \cdot cos^2(\beta/2) = \; cos\alpha \cdot sin\beta$$

The esEPI-BUDA sequence also contains a small gradient with ½ phase-encoding blip area ($G_y$) prior to the second echo-train so that the two k-space trajectories are interleaved (Figure 4B) for effective joint reconstruction.

BUDA reconstruction:

Figure 12 shows the steps involved in 3D esEPI-BUDA image reconstruction. Each echo-train dataset first underwent SENSE reconstruction using TOPUP in FSL to estimate a field map *E*, which was subsequently incorporated to jointly reconstruct the data from both echo-trains according to:

$$argmin_{\boldsymbol{I}} \sum_t \|F_t ECI_t - d_t\|_2^2 + \lambda \|\mathcal{H}(I)\|_*$$

where *t* is the echo-train index (1 or 2), $F_t$ is the Fourier operator, *C* is the coil sensitivity, and $I_t$ and $d_t$ are the targeted distortion-corrected image and the k-space data for the $t^{th}$ echo-train, respectively. The constraint $\| \mathcal{H}(I)\|_*$ enforces low-rank prior on the block-Hankel representation of the two datasets, and $\lambda$ is the parameter to tune the weight of structured low rank regularization.

Experiments:

The 3D esEPI-BUDA sequence was implemented on a GE MR750 3T scanner. 3D fMRI experiments were performed on healthy human brains using a 32-channel head coil with a visual stimulation paradigm. The paradigm contained six 48-s blocks with 24-s stimulus followed by 24-s rest. The imaging parameters were: TR/TE = 75/30ms, volume TR = 2.4s, $\alpha \approx \beta$ = 15° (Ernst angle), FOV = 220×220×128mm$^3$, acquisition matrix = 72×72×32, spatial resolution = 3.1×3.1×4.0mm$^3$, and under-sampling factor along the in-slab phase-encoding direction = 2 (i.e., the length of each echo-train = 36; total length of two echo-trains = 72). For comparison, images over the same volume were also acquired using 3D fully-sampled EPI (flip angle = 15°) with blip-up and blip-down acquisitions separately.

**Results:**

Figure 13 displays a set of representative whole-brain images obtained using 3D esEPI-BUDA, illustrating excellent image quality. The BUDA reconstruction produced images with noticeably reduced geometric distortion, especially at the frontal lobe, when compared to the 3D fully-sampled EPI images (Figure 14A-D). Results from the fMRI experiment are illustrated in Figure 15A where six contiguous activation maps from 3D esEPI-BUDA are overlaid on the corresponding T1-weighted images. fMRI activations were observed in the visual cortex, as expected. Figure 15B shows the time evolution of the BOLD signal change (~4.0%), which is comparable with that of 3D fully-sampled EPI.

An exemplary pulse sequence - 3D esEPI-BUDA has been demonstrated herein that accomplished distortion correction with 3D whole-brain coverage by acquiring the blip-up and blip-down datasets in a single shot without increasing the scan time and without being subject to inter-shot motion. Although the demonstration was for 3D fMRI, the same strategy can be extended to other EPI-based applications such as 2D fMRI, 2D/3D diffusion, and 2D/3D perfusion imaging. Also, while the above description uses fMRI as an example, the methods described herein can be applied for diffusion imaging, perfusion imaging, and any other MRI applications where echo-train-based echo planar imaging acquisitions are employed. Other embodiments of systems, methods and components constructed in accordance with the principles herein are contemplated as well.

**Claims**

**1.** A method for using an MRI system electrically coupled to a computing device, the method comprising:

generating, via the MRI system, an echo-shifted echo-planar imaging with blip up/down acquisition ("esEPI-BUDA") pulse sequence comprising a first radiofrequency ("RF") pulse and a second RF pulse, the first RF pulse and the second RF pulse are followed by successive first and second echo-trains such that the first and the second echo-trains have opposite phase-encoding blip gradient polarities to traverse echo planar imaging ("EPI") k-space in a reversed order;
generating, via the MRI system, a plurality of echo-shifting gradients applied along a direction perpendicular to the imaging plane by:

generating, via the MRI system, a first echo-shifting gradient with an area of G' and thereby dephasing transverse magnetization from the first RF pulse;
after the transverse magnetization is dephased, generating, via the MRI system, the second RF pulse and thereby exciting the stored longitudinal magnetization;
after the second RF pulse is generated, generating, via the MRI system, a second echo-shifting gradient with an area of -G and thereby dephasing transverse magnetization from the second RF pulse and rephasing a signal produced by the first RF pulse; and
after the signal produced by the first RF pulse is acquired, generating, via the MRI system, a third echo-shifting gradient with an area of G and thereby dephasing transverse magnetization from the first RF pulse and rephasing a signal produced by the second RF pulse;

in response to the pulse sequence being generated, the MRI system acquiring two interleaved k-space datasets within a single shot; and
correcting image distortion, via the MRI system, based on the two acquired interleaved k-space datasets.

**2.** The method of claim 1, wherein the first RF pulse has a flip angle of $\alpha$ and the second RF pulse has a flip angle of $\beta$, with $\alpha$ and $\beta$ satisfying the following condition:

$$\sin\alpha \cdot \cos^2(\beta/2) = \cos\alpha \cdot \sin\beta.$$

**3.** The method of claim 1, where G' = $G - A$ with $A$ being the absolute value of the area of a slice-refocusing gradient associated with the first or the second RF pulse.

**4.** The method of claim 1, further comprising:
acquiring, via the first echo-train with blip-up phase-encoding, the rephased signal produced by the first RF pulse.

**5.** The method of claim 1, further comprising:
acquiring, via the second echo-train with blip-down phase-encoding, the rephased signal produced by the second RF pulse.

**6.** The method of claim 1, wherein the esEPI-BUDA pulse sequence further comprises:
after acquiring the rephased signal produced by the first RF pulse and before acquiring the rephased signal produced by the second RF pulse, generating, via the MRI system, a gradient having one half (½) of an individual phase-encoding blip gradient area ($G_y$).

**7.** The method of claim 1, further comprising:
under-sampling, via the MRI system, k-space data from the first echo-train and the second echo-train and thereby shortening a length of each of the first echo-train and the second echo-train.

**8.** The method of claim 1, wherein correcting image distortion based on the two acquired k-space datasets further comprises:

generating, via the MRI system, dynamic maps of a main magnetic field; and
incorporating, via the MRI system, the dynamic maps of the main magnetic field into a forward joint parallel imaging reconstruction model with Hankel structured low-rank constraints and thereby correcting image geometric distortion.

**9.** The method of claim 1, wherein correcting image distortion based on the two acquired k-space datasets further comprises:
combining, via the MRI system, the two acquired k-space datasets and thereby improving the image signal-to-noise ratio.

**10.** A non-transitory computer-readable medium having stored thereon program instructions that upon execution by a processor, cause performance of the method according to any one of the claims 1 to 9, upon execution by an MRI system.

## Patentansprüche

**1.** Verfahren zur Verwendung eines MRT-Systems, das elektrisch mit einer Rechenvorrichtung gekoppelt ist, das Verfahren umfassend:

Erzeugen, über das MRT-System, einer echoverschobenen Echo-Planar-Bildgebung mit Blip-Up-Down-Akquisition ("esEPI-BUDA") Pulssequenz, umfassend einen ersten Hochfrequenzpuls ("RF-Puls") und einen zweiten RF-Puls, wobei auf den ersten RF-Puls und den zweiten RF-Puls aufeinanderfolgende erste und zweite Echozüge folgen , sodass die ersten und zweiten Echozüge entgegengesetzte Polaritäten des Phasenkodier-Blip-Gradienten aufweisen, um den Echo-Planar-Bildgebung ("EPI") k-Raum in umgekehrter Abtastreihenfolge zu durchlaufen;
Erzeugen, über das MRT-System, einer Vielzahl von Echoverschiebegradienten die entlang einer Richtung senkrecht zur Bildebene angewendet werden, durch:

Erzeugen, über das MRT-System, eines ersten Echoverschiebegradienten mit einer Fläche von G', wodurch die transversale Magnetisierung vom ersten RF-Puls dephasiert wird;
nach der Dephasierung der transversalen Magnetisierung, Erzeugen, über das MRT-System, des zweiten RF-Pulses und dadurch Anregen der gespeicherten longitudinalen Magnetisierung;

nach dem Erzeugen des zweiten RF-Pulses, Erzeugen, über das MRT-System, eines zweiten Echoverschiebegradienten mit einer Fläche von -G und dadurch Dephasierung der transversalen Magnetisierung vom zweiten RF-Puls und Rephasierung eines durch den ersten RF-Puls hergestellten Signals; und nachdem das durch den ersten RF-Puls erzeugte Signal erfasst wurde, Erzeugen, über das MRT-System, eines dritten Echoverschiebegradienten mit einer Fläche von G und dadurch Dephasierung der transversalen Magnetisierung vom ersten RF-Puls und Rephasierung eines durch den zweiten RF-Puls hergestellten Signals;

als Antwort auf die erzeugte Pulssequenz das MRT-System erfassend zwei verschränkte k-Raum Datensätze innerhalb eines Einzelschusses; und
Korrigieren der Bildverzerrung, über das MRT-System, auf der Basis der beiden erfassten verschränkten k-Raum Datensätze.

**2.** Verfahren nach Anspruch 1, wobei der erste RF-Puls einen Kippwinkel von $\alpha$ und der zweite RF-Puls einen Kippwinkel von $\beta$ aufweist, wobei $\alpha$ und $\beta$ die folgende Bedingung erfüllen:

$$sin\,\alpha \cdot cos^2(\beta/2) = \; cos\alpha \cdot sin\beta.$$

**3.** Verfahren nach Anspruch 1, wobei *G' = G - A,* wobei *A* der Absolutwert der Fläche des Schichtrephasierungsgradienten ist, verbunden mit dem ersten oder dem zweiten RF-Puls.

**4.** Verfahren nach Anspruch 1, ferner umfassend:
Erfassen des durch den ersten RF-Puls hergestellten neu phasengeregelten Signals über den ersten Echozug mit Blip Up Phasenkodierung.

**5.** Verfahren nach Anspruch 1, ferner umfassend:
Erfassen des durch den zweiten RF-Puls hergestellten neu phasengeregelten Signals über den zweiten Echozug mit Blip Down Phasenkodierung.

**6.** Verfahren nach Anspruch 1, wobei die esEPI-BUDA-Pulssequenz ferner umfasst:
nach dem Erfassen des durch den ersten RF-Puls hergestellten neu phasengeregelten Signals und vor dem Erfassen des durch den zweiten RF-Puls hergestellten neu phasengeregelten Signals das Erzeugen eines Gradienten mit der Hälfte (½) der Fläche des Phasenkodier-Blip-Gradienten ($G_y$) über das MRT-System.

**7.** Verfahren nach Anspruch 1, ferner umfassend:
Unterabtastung, über das MRT-System, von k-Raum Daten aus dem ersten Echozug und dem zweiten Echozug und dadurch Verkürzung der Länge des ersten Echozuges und des zweiten Echozuges.

**8.** Verfahren nach Anspruch 1, wobei das Korrigieren der Bildverzerrung auf der Grundlage der beiden erfassten k-Raum-Datensätze ferner umfasst:

Erzeugen, über das MRT-System, dynamischer Karten des Hauptmagnetfelds; und
Einbindung, über das MRT-System, der dynamischen Karten des Hauptmagnetfelds in ein gemeinsames Vorwärtsparallelbildgebungsrekonstruktionsmodell mit Hankel-strukturierten Niedrigrang-Nebenbedingungen und dadurch Korrigieren der geometrischen Bildverzerrung.

**9.** Verfahren nach Anspruch 1, wobei das Korrigieren der Bildverzerrung auf der Grundlage der beiden erfassten k-Raum-Datensätze ferner umfasst:
Kombinieren, über das MRT-System, der beiden erfassten k-Raum-Datensätze und dadurch Verbessern des Bild-Signal-Rausch-Verhältnisses.

**10.** Nichtflüchtiges, computerlesbares Medium, auf dem Programminstruktionen gespeichert sind, die bei Ausführung durch einen Prozessor die Durchführung des Verfahrens gemäss einem der Ansprüche 1 bis 9 bei Ausführung durch ein MRT-System bewirken.

## Revendications

1. Procédé d'utilisation d'un système IRM couplé électriquement à un dispositif informatique, le procédé comprenant :

    générer, via le système IRM, une imagerie écho planaire à décalage d'écho avec acquisition blip up blip down (« esEPI-BUDA ») séquence d'impulsions comprenant une première impulsion radiofréquence (« RF ») et une deuxième impulsion RF, la première impulsion RF et la deuxième impulsion RF étant suivies par des premier et deuxième trains d'échos successifs de telle sorte que les premier et deuxième trains d'échos aient des polarités opposées des gradients blip de codage de phase pour traverser l'espace k de l'imagerie écho planaire (« EPI ») dans un ordre inversé ;
    générer, via le système IRM, une pluralité de gradients de décalage d'écho appliqués le long d'une direction perpendiculaire au plan d'imagerie en :

        générer, via le système IRM, un premier gradient de décalage d'écho avec une aire de G' et réaliser un déphasage de l'aimantation transversale à partir de la première impulsion RF ;
        une fois l'aimantation transversale déphasée, générer, via le système IRM, la deuxième impulsion RF et exciter ainsi l'aimantation longitudinale stockée ;
        après la génération de la deuxième impulsion RF, générer, via le système IRM, le deuxième gradient de décalage d'écho avec une aire de -G et réaliser un déphasage de l'aimantation transversale de la deuxième impulsion RF et un rephasage du signal produit par la première impulsion RF ; et
        après l'acquisition du signal produit par la première impulsion RF, générer, via le système IRM, le troisième gradient de décalage d'écho avec une aire de G et réaliser un déphasage de l'aimantation transversale de la première impulsion RF et un rephasage du signal produit par la deuxième impulsion RF ;

    en réponse à la séquence d'impulsions générée, le système IRM acquiert deux jeux de données en espace k entrelacés en un tir unique ; et
    corriger la distorsion d'image, via le système IRM, sur la base des deux jeux de données en espace k entrelacés acquis.

2. Procédé selon la revendication 1, dans lequel la première impulsion RF a un angle de bascule de $\alpha$ et la deuxième impulsion RF a un angle de bascule de $\beta$, $\alpha$ et $\beta$ satisfaisant la condition suivante :

$$sin\,\alpha \cdot cos^2(\beta/2) = \ cos\alpha \cdot sin\beta.$$

3. Procédé selon la revendication 1, dans lequel $G' = G - A$ , où $A$ est la valeur absolue de l'aire du gradient de refocalisation de coupe associée à la première ou à la deuxième impulsion RF.

4. Procédé selon la revendication 1, comprenant en outre :
l'acquisition, via le premier train d'échos avec codage de phase blip up, du signal rephasé fabriqué par la première impulsion RF.

5. Procédé selon la revendication 1, comprenant en outre :
l'acquisition, via le deuxième train d'échos avec codage de phase blip down, du signal rephasé produit par la deuxième impulsion RF.

6. Procédé selon la revendication 1, dans lequel la séquence d'impulsions esEPI-BUDA comprend en outre :
après l'acquisition du signal rephasé produit par la première impulsion RF et avant l'acquisition du signal rephasé produit par la deuxième impulsion RF, la génération, via le système IRM, d'un gradient ayant la moitié (½) de l'aire du gradient blip de codage de phase individuel ($G_y$ ).

7. Procédé selon la revendication 1, comprenant en outre :
sous-échantillonner, via le système IRM, les données de l'espace k provenant du premier train d'échos et du deuxième train d'échos, raccourcissant ainsi la longueur de chacun de premier train d'échos et de deuxième train d'échos.

8. Procédé selon la revendication 1, dans lequel la correction de la distorsion d'image sur la base des deux ensembles de données de l'espace k acquis comprend en outre :

générer, via le système IRM, des cartes dynamiques du champ magnétique principal ; et
intégrer, via le système IRM, les cartes dynamiques du champ magnétique principal dans un modèle de reconstruction conjointe en imagerie parallèle avec des contraintes de faible rang structurées de Hankel, et réaliser ainsi la correction de distorsion géométrique de l'image.

**9.** Procédé selon la revendication 1, dans lequel la correction de la distorsion d'image sur la base des deux ensembles de données de l'espace k acquis comprend en outre :
combiner, via le système IRM, les deux ensembles de données en espace k acquis et réaliser ainsi une amélioration du rapport signal sur bruit de l'image.

**10.** Support lisible par ordinateur non transitoire sur lequel sont stockées des instructions de programme qui, lorsqu'elles sont exécutées par un processeur, provoquent la mise en œuvre du procédé selon l'une quelconque des revendications 1 à 9, lors de leur exécution par un système IRM.

Network
214
Computing Device
200
Environment
100
Computing Device
200a
MRI System
105

FIG. 1

200
212
Processor(s)
202
Communication Interface
204
Output Interface
208
Display
210
Data Storage
206
Executable Instructions
218
Tablet
216a
Personal Computer
216b
Network
214
Mobile Computing Device
216d
Laptop Computer
216c

FIG. 2

300
305
Generating, via the MRI system, an echo-shifted echo-planar imaging with blip up/down acquisition ("esEPI-BUDA") pulse sequence comprising a first radiofrequency ("RF") pulse and a second RF pulse, the first RF pulse followed by a first echo-train that is interleaved with the first and the second RF pulses, and the second RF pulse followed by a second echo-train such that the first and the second echo-trains have opposite phase-encoding blip gradient polarities to traverse echo planar imaging ("EPI") k-space in a reversed order
310
In response to the pulse sequence being generated, the MRI system acquiring two k-space datasets within a single shot
315
Correcting image distortion, via the MRI system, based on the two acquired k-space datasets

FIG. 3

TE
TE
α
β
RF
2A
-A
G-A
2A
-A
-G
3D phase-encoding
G
Gz
Gx
Gy
½ blip

FIG. 4A

Ky
Kx
Ky
Kx

FIG. 4B

FIG. 14A FIG. 14B FIG. 14C FIG. 14D

SENSE Echo-train1
SENSE Echo-train2
Distortion-corrected
BUDA
FSL-TOPUP
50 Hz
-50 Hz
Field Map
BUDA Recon
Structured Low-rank
Constraint
$argmin_I \sum_t \|U_t F_t E C I_t - d_t\|_2^2 + \lambda \|\mathcal{H}(I)\|_*$

FIG. 5

A
B
D
E
TOPUP in FSL
esEPI-BUDA
C
G
F

FIG. 6

A
B
D
E
TOPUP in FSL
esEPI-BUDA
C
G
F

FIG. 9

1
2
10
11
12
20
111
112
120
Δt = 2.4s
Time
B0 field map (Hz)
Time points
Frontal lobe
Occipital lobe
Spatial dislocation (mm)
Time Points

FIG. 7A

FIG. 7B

FIG. 7C

FIG. 8A

FIG. 8B

FIG. 8C

A
3D fully-sampled EPI with blip-up acquisition
B
3D fully-sampled EPI with blip-down acquisition
C
3D EPI TOPUP
D
3D esEPI-BUDA
20
10
0

FIG. 10

signal change(%)
Time(s)
4
3
2
1
0
-1
0
20
40
60

FIG. 11A

FIG. 11B

FIG. 11C

FIG. 11D

SENSE Echo1
SENSE Echo2
Distortion-corrected
BUDA
FSL-TOPUP
BUDA Recon
100 Hz
-100 Hz
Field Map
Structured Low-rank
Constraint
$argmin_I \sum_t \|F_t E C I_t - d_t\|_2^2 + \lambda \|\mathcal{H}(I)\|_*$

FIG. 12

FIG. 13

3D esEPI-BUDA
3D fully-sampled EPI with blip-up acquisition
3D fully-sampled EPI with blip-down acquisition
20
10
0

FIG. 15A

3D esEPI-BUDA
3D fully-sampled EPI with blip-up acquisition
3D fully-sampled EPI with blip-down acquisition
Signal changes(%)
Time points

FIG. 15B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Highly efficient MRI through multi-shot echo planar imaging. **CONGYU LIAO et al.** arXiv:1908.00280. Cornell University Library, 02 August 2019